(19) Europäisches Patentamt
European Patent Office
Office européen des brevets

(11) Veröffentlichungsnummer: **0 202 566**
**A2**

(12) # EUROPÄISCHE PATENTANMELDUNG

(21) Anmeldenummer: **86106398.0**

(22) Anmeldetag: **12.05.86**

(51) Int. Cl.[4]: **C 07 D 249/08**
A 01 N 43/653

(30) Priorität: **22.05.85 JP 108436/85**

(43) Veröffentlichungstag der Anmeldung:
**26.11.86 Patentblatt 86/48**

(84) Benannte Vertragsstaaten:
**AT BE CH DE FR GB IT LI NL**

(71) Anmelder: **NIHON TOKUSHU NOYAKU SEIZO K.K.**
**No.4, 2-chome, Nihonbashi Honcho Chuo-ku**
**Tokyo 103(JP)**

(72) Erfinder: **Kurahashi, Yoshio**
**47-15, Oya-machi**
**Hachioji-shi Tokyo(JP)**

(72) Erfinder: **Kume, Toyohiko**
**6-7-8, Asahigaoka**
**Hino-shi Tokyo(JP)**

(72) Erfinder: **Isono, Kunihiro**
**2-32-4, Asahigaoka**
**Hino-shi Tokyo(JP)**

(72) Erfinder: **Matsumoto, Noboru**
**39-15, Namiki-cho**
**Hachioji-shi Tokyo(JP)**

(72) Erfinder: **Izumi, Tetsuji**
**39-15, Namiki-cho**
**Hachioji-shi Tokyo(JP)**

(74) Vertreter: **Schumacher, Günter, Dr. et al,**
**c/o Bayer AG Konzernverwaltung RP Patentabteilung**
**D-5090 Leverkusen 1 Bayerwerk(DE)**

(54) **Phenoxyalkyltriazole.**

(57) Neue Phenoxyalkyltriazole der Formel

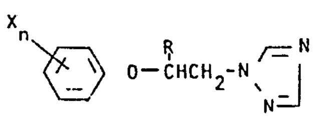

(I)

in welcher

R für Alkyl steht,

X für Halogen, Alkyl, Alkoxy, Alkylthio, Halogenalkyl, Halogenalkoxy, Halogenalkylthio, Nitro und/oder Cyano steht und

n für die Zahlen 0, 1, 2, 3, 4 oder 5 steht,

sowie deren Säureadditions-Salze,

mehrere Verfahren zur Herstellung der neuen Stoffe und deren Verwendung als Fungizide.

Croydon Printing Company Ltd

0202566

NIHON TOKUSHU NOYAKU SEIZO K.K.
Tokyo / Japan

Dü/Kü-c
Ib

# Phenoxyalkyltriazole

Die vorliegende Erfindung betrifft neue Phenoxyalkyltriazole, mehrere Verfahren zu ihrer Herstellung und ihre Verwendung als Fungizide.

Es ist bereits bekannt, daß bestimmte 1,2,4-Triazol-Derivate fungizide Eigenschaften besitzen (vgl. GB-PS 1 563 199 und JP-OS 33 675/1977). So kann z.B. 1-(4-Chlorphenoxy)-1-(1,2,4-triazol-1-yl)-ethan zur Bekämpfung von Pilzen verwendet werden. In manchen Fällen ist die Wirkung dieses Stoffes jedoch nicht ganz befriedigend.

Es wurden nun neue Phenoxyalkyltriazole der Formel

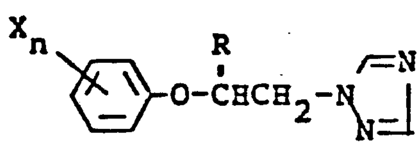

(I)

in welcher

R für Alkyl steht,

X für Halogen, Alkyl, Alkoxy, Alkylthio, Halogenalkyl, Halogenalkoxy, Halogenalkylthio, Nitro und/oder Cyano steht und

n für die Zahlen 0, 1, 2, 3, 4 oder 5 steht,

sowie deren Säureadditionssalze gefunden.

Die erfindungsgemäßen Stoffe weisen ein asymmetrisch substituiertes Kohlenstoffatom auf und können deshalb als Racemate oder auch in den einzelnen optischen Isomeren-Formen vorliegen. Die Erfindung betrifft sowohl die Racemate als auch die einzelnen optischen Antipoden und deren Gemische.

Weiterhin wurde gefunden, daß sich Phenoxyalkyltriazole der Formel (I) sowie deren Säureadditions-Salze herstellen lassen, indem man

a) Verbindungen der Formel

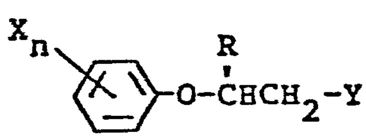

(II)

in welcher

R, X und n die oben angegebene Bedeutung haben und

Y für Halogen oder eine Gruppe der Formel $-SO_2R^1$ steht, worin

$R^1$ für Alkyl oder gegebenenfalls substituiertes Aryl steht,

mit Verbindungen der Formel

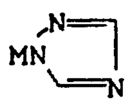 (III)

in welcher

M für Wasserstoff oder ein Alkalimetallatom steht,

gegebenenfalls in Gegenwart eines inerten Verdünnungsmittels und gegebenenfalls in Gegenwart eines Säurebindemittels umsetzt,

oder

b) Verbindungen der Formel

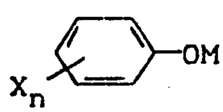 (IV)

in welcher

X, M und n die oben angegebene Bedeutung haben,

mit Verbindungen der Formel

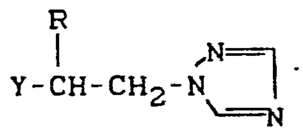

(V)

in welcher

R und Y die oben angegebene Bedeutung haben,

gegebenenfalls in Gegenwart eines inerten Lösungsmittels sowie gegebenenfalls in Gegenwart eines Säurebindemittels umsetzt, wobei die Verbindungen der Formel (I) auch in Form ihrer Säureadditions-Salze eingesetzt werden können,

und gegebenenfalls anschließend an die so erhaltenen Stoffe der Formel (I) eine Säure addiert.

Schließlich wurde gefunden, daß die neuen Phenoxyalkyltriazole der Formel (I) sowie deren Säureadditions-Salze sehr gute fungizide Eigenschaften besitzen.

Überraschenderweise zeigen die erfindungsgemäßen Stoffe eine wesentlich stärkere fungizide Wirkung als die konstitutionell ähnlichsten Stoffe, die aus dem Stand der Technik bekannt sind. So übertreffen die erfindungsgemäßen Stoffe z.B. das 1-(4-Chlorphenoxy)-1-(1,2,4-triazol-1-yl)-ethan bezüglich der fungiziden Eigenschaften.

Bevorzugte Phenoxyalkyltriazole sind diejenigen Stoffe der Formel (I), in denen

R für Alkyl mit 2 bis 5 Kohlenstoffatomen steht,

X gleichartig oder verschieden ist und für Fluor, Chlor, Brom, Alkyl mit 1 bis 4 Kohlenstoffatomen, Alkoxy mit 1 bis 4 Kohlenstoffatomen, Alkylthio mit 1 bis 4 Kohlenstoffatomen, gegebenenfalls durch Fluor und/oder Chlor substituiertes Alkyl mit 1 bis 3 Kohlenstoffatomen, gegebenenfalls durch Fluor und/oder Chlor substituiertes Alkoxy mit 1 bis 3 Kohlenstoffatomen, gegebenenfalls durch Fluor und/oder Chlor substituiertes Alkylthio mit 1 bis 3 Kohlenstoffatomen, Nitro und/oder Cyan steht und

n für die Zahlen 1, 2 oder 3 steht.

Besonders bevorzugte Phenoxyalkyltriazole der Formel (I) sind diejenigen, in denen

R für Ethyl, n-Propyl, Isopropyl, n-Butyl, Isobutyl, sec-Butyl oder t-Butyl steht,

X gleichartig oder verschieden ist und für Fluor, Chlor, Methyl, Ethyl, Methoxy, Ethoxy, Methylthio, Trifluormethyl, Trifluormethoxy, Trifluormethylthio, Nitro und/oder Cyano steht und

n für 1 oder 2 steht.

Bevorzugte erfindungsgemäße Verbindungen sind auch Additionsprodukte aus Säuren und denjenigen Phenoxyalkyltriazolen der Formel (I), in denen R, X und n die Bedeutungen haben, die bereits für diese Substituenten und für den Index n als bevorzugt genannt wurden.

Zu den Säuren, die addiert werden können, gehören vorzugsweise Halogenwassertoffsäuren, wie z.B. die Chorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure, sowie Sulfonsäuren, wie z.B. p-Toluolsulfonsäure und 1,5-Naphthalindisulfonsäure.

Als Beispiele für Phenoxyalkyltriazole der Formel (I) seien speziell genannt:

1-[2-(2,4-Dichlorphenoxy)-n-butyl]-1H-1,2,4-triazol und
1-[2-(2,4-Dichlorphenoxy)-n-pentyl]1H-1,2,4-triazol.

Die erfindungsgemäßen Stoffe lassen sich nach dem oben angegebenen Verfahren (a) herstellen, das durch das folgende Formelschema veranschaulicht werden kann:

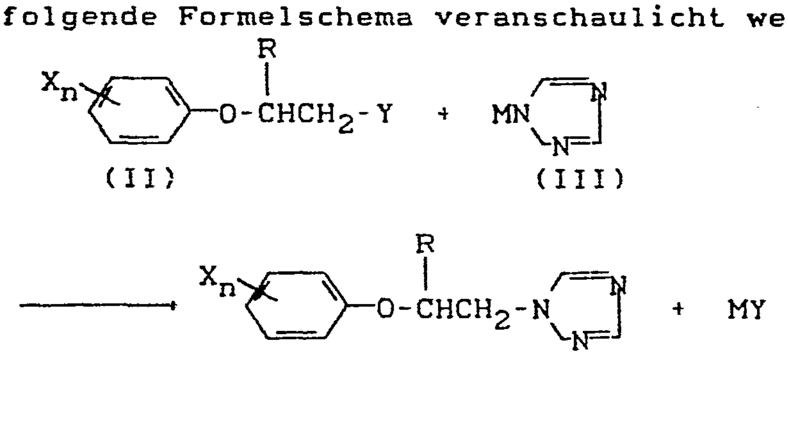

(II) (III)

(In den vorstehenden Formeln haben R, X, n, Y und M die oben angegebenen Bedeutungen).

Wenn beispielsweise 1-[1-(Chlormethyl)-butoxy]-2,4-dichlorbenzol und 1,2,4-Triazol als Ausgangsstoffe eingesetzt werden, läßt sich das vorstehende Verfahren (a) durch die folgende Gleichung darstellen:

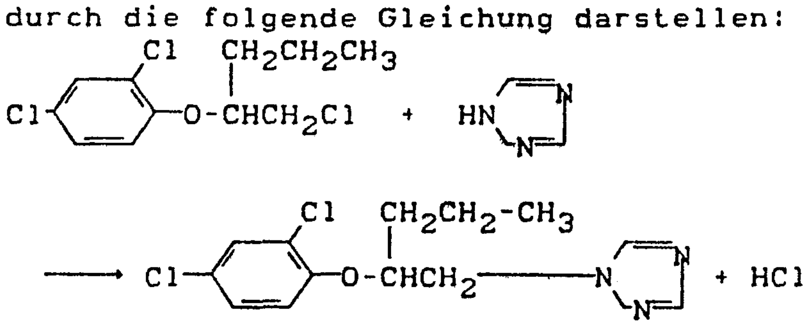

Die erfindungsgemäßen Stoffe lassen sich auch nach dem oben angegebenen Verfahren (b) herstellen, das durch das folgende Formelschema veranschaulicht wird:

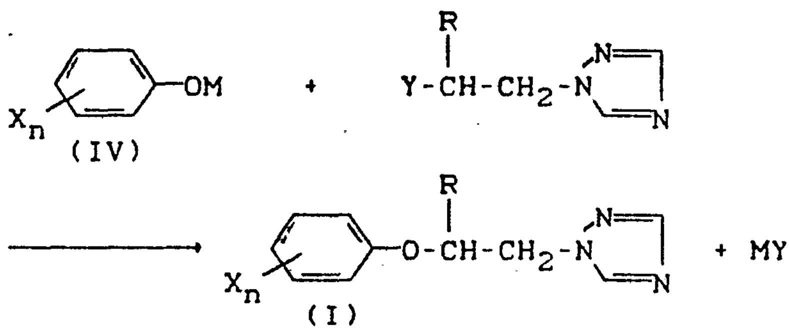

(IV) (V) (I)

Wenn beispielsweise 2,4-Dichlorphenol-Natrium und 2-Chlor-1-(1,2,4-triazol-1-yl)-butan als Ausgangsstoffe eingesetzt werden, läßt sich das oben angegebene Verfahren (b) durch folgende Gleichung darstellen:

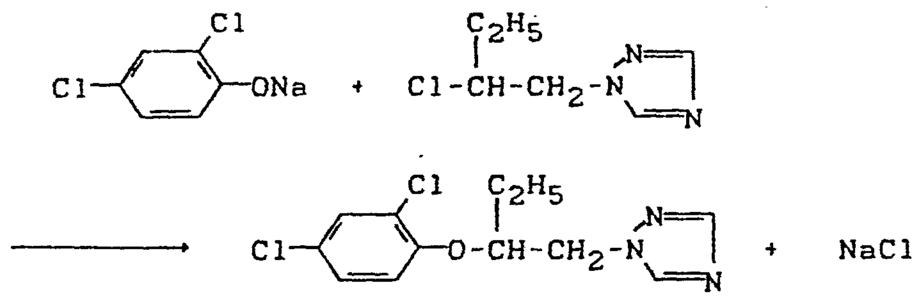

Die bei dem erfindungsgemäßen Verfahren (a) als Ausgangsstoffe benötigten Verbindungen sind durch die Formel (II) definiert. In dieser Formel haben R, X und n vorzugsweise diejenigen Bedeutungen, die bereits im Zusammenhang mit der Beschreibung der erfindungsgemäßen Stoffe der Formel (I) vorzugsweise für diese Reste bzw. für diesen Index genannt wurden. Der Rest Y steht vorzugsweise für Chlor, Brom oder einen Rest der Formel $-OSO_2R^1$, worin $R^1$ für Methyl, Phenyl oder 4-Methylphenyl steht.

Zu den Verbindungen der Formel (II) zählen sowohl neue als auch bekannte Verbindungen:

Als Beispiele für die bekannten Verbindungen seien
1-[1-(Brommethyl)propoxy]-4-methylbenzol und
1-[1-(Brommethyl)propoxy]-4-methoxybenzol angeführt, die beispielsweise in J. Med. Chem. 11 (1968), Seiten 1190-1201, beschrieben sind.

Zu Beispielen für die neuen Verbindungen zählen
1-Chlor-2-[1-(chlormethyl)butoxy]benzol,
1-Chlor-4-[1-(chlormethyl)propoxy]benzol
1-Chlor-4-[1-(chlormethyl)butoxy]benzol,
1-[1-(Chlormethyl)propoxy]-2,4-dichlorbenzol,

1-[1-(Chlormethyl)butoxy]-2,4-dichlorbenzol,
1-[1-(Chlormethyl)-2-methylpropoxy]-2,4-dichlorbenzol,
1-[1-(Chlormethyl)pentyloxy]-2,4-dichlorbenzol,
1-[1-(Chlormethyl)-2,2-dimethylpropoxy]-2,4-dichlorbenzol,
1-[1-(Chlormethyl)propoxy]-2,6-dichlorbenzol,
1-[1-(Chlormethyl)propoxy]-4-(trifluormethyl)-benzol,
1-[1-(Chlormethyl)propoxy]-4-(trifluormethoxy)-benzol,
1-[1-(Chlormethyl)propoxy]-2-chlor-4-(trifluormethoxy)benzol
1-[1-(Chlormethyl)propoxy]-2,6-dichlor-4-(trifluormethoxy)benzol,
1-[1-(Chlormethyl)propoxy]-2,6-dichlor-4-(trifluormethylthio)benzol,
1-[1-(Chlormethyl)propoxy]-4-nitrobenzol und
1-[1-(Chlormethyl)propoxy]-4-cyanobenzol.

Diese Verbindungen sind Beispiele für Verbindungen der Formel (II), in denen Y Chlor ist.

Daneben können auch die entsprechenden Verbindungen der Formel (II), in denen

Y Brom, $-SO_2CH_3$, $-OSO_2$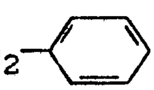 oder $-OSO_2$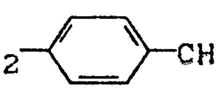$CH_3$

ist,

einbezogen werden.

Diejenigen Verbindungen der Formel (II), in denen Y für Halogen steht, lassen sich nach dem Verfahren (c) herstellen, das durch das folgende Formelschema veranschaulicht wird:

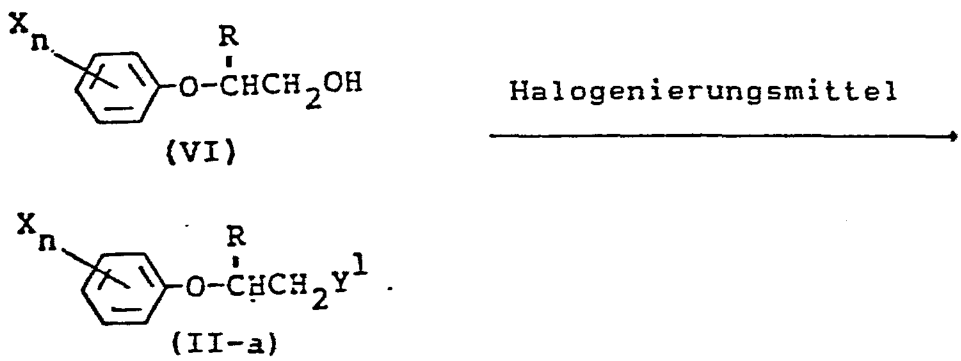


(VI)

(II-a)

In den Formeln (VI) und (II-a) haben R, X und n die oben angegebenen Bedeutungen, und $Y^1$ steht für Halogen, vorzugsweise für Chlor oder Brom.

Wenn beispielsweise 2-(2,4-Dichlorphenoxy)-n-pentanol und Thionylchlorid als Ausgangsstoffe eingesetzt werden, läßt sich das vorstehende Verfahren (c) durch die folgende Gleichung darstellen:

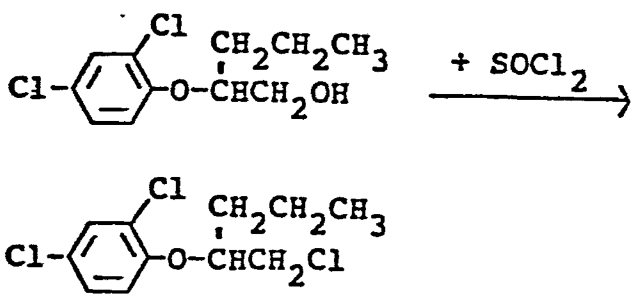

Diejenigen Verbindungen der Formel (II), in denen Y für den Rest der Formel $-OSO_2R^1$ steht, lassen sich nach dem Verfahren (d) herstellen, das durch das folgende Formelschema veranschaulicht wird:

Verfahren (d):

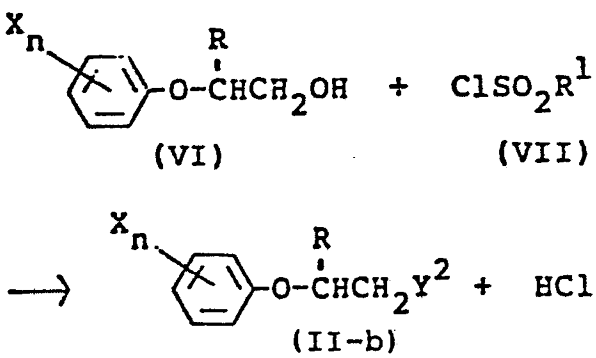

(VI) (VII) (II-b)

In den Formeln (VI), (VII) und (II-b) haben R, $R^1$, X und n die oben angegebenen Bedeutungen, und $Y^2$ steht für den Rest der Formel $-OSO_2R^1$, wobei $R^1$ vorzugsweise für Methyl, Phenyl oder 4-Methyl-phenyl steht.

Wenn beispielsweise 2-(2,4-Dichorphenoxy)-butanol und Methansulfonylchlorid als Ausgangsstoffe eingesetzt werden, läßt sich das vorstehende Verfahren (d) durch die folgende Gleichung darstellen:

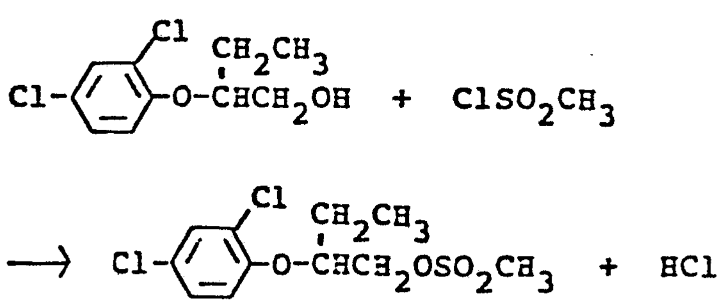

Die bei den Verfahren (c) und (d) als Ausgangsstoffe benötigten Verbindungen sind durch die Formel (VI) definiert. In dieser Formel haben die Reste R und X sowie der Index n vorzugsweise diejenigen Bedeutungen, die bereits im Zusammenhang mit der Beschreibung der erfindungsgemäßen Stoffe der Formel (I) vorzugsweise für diese Reste bzw. diesen Index genannt wurden.

Die meisten der Verbindungen der Formel (VI) sind neue Verbindungen. Als Beispiele seien genannt:

2-(2-Chlorphenoxy)-n-pentanol,
2-(4-Chlorphenoxy)-n-butanol,
2-(4-Chlorphenoxy)-n-pentanol,
2-(2,4-Dichlorphenoxy)-n-butanol,
2-(2,4-Dichlorphenoxy)-n-pentanol,
2-(2,4-Dichlorphenoxy)-4-methyl-n-butanol,
2-(2,4-Dichlorphenoxy)-n-hexanol,
2-(2,4-Dichlorphenoxy)-3,3-dimethyl-n-butanol,
2-(2,6-Dichlorphenoxy)-n-butanol,
2-[4-(Trifluormethyl)phenoxy]-n-butanol,
2-[4-(Trifluormethoxy)phenoxy]-n-butanol,
2-[2-Chlor-4-(trifluormethoxy)phenoxy]-n-butanol,
2-[2,6-Dichlor-4-(trifluormethoxy)phenoxy]-n-butanol,
2-[2,6-Dichlor-4-(trifluormethylthio)phenoxy]-n-butanol,
2-(4-Nitrophenoxy)-n-butanol und
2-(4-Cyanophnoxy)-n-butanol.

Die Verbindungen der Formel (VI) sind, - wie schon oben erwähnt -, teilweise bekannt. Sie lassen sich nach dem Verfahren (e) herstellen, daß durch das folgende Formel-

schema veranschaulicht wird:

<u>Verfahren (e)</u>

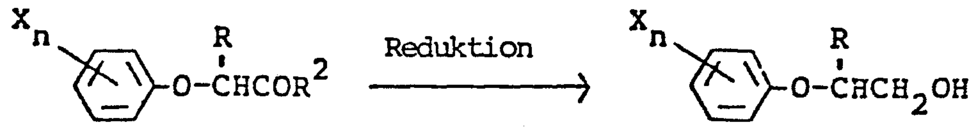


(VIII) (VI)

In den Formeln (VI) und VIII) haben R, X und n die oben angegebenen Bedeutungen, und $R^2$ steht für Halogen, Hydroxy oder $OR^1$, wobei $R^1$ die oben angegebenen Bedeutungen hat.

Wenn beispielsweise Ethyl-2-(2,4-dichlorphenoxy)butyrat und Lithiumaluminiumhydrid als Reduktionsmittel eingesetzt werden, läßt sich das vorstehene Verfahren (e) durch die folgende Gleichung darstellen:

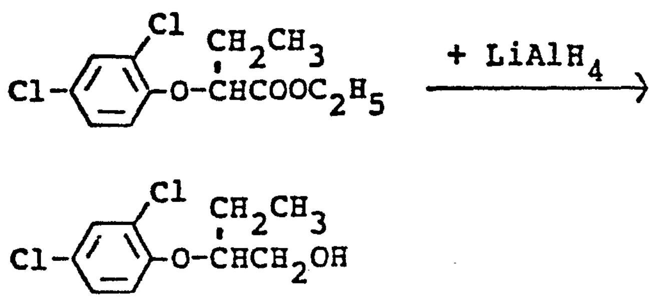


Die Verbindungen der Formel (VI) lassen sich auch nach dem Verfahren (f) herstellen, das durch das folgende Formelschema veranschaulicht wird:

Verfahren (f)

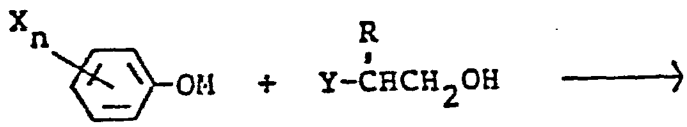

(IV) (IX)

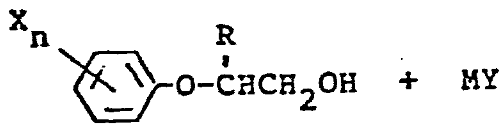

(VI)

In den Formeln (IV), (VI) und (IX) haben R, X, Y, M und n die oben angegebenen Bedeutungen.

Wenn beispielsweise 2,4-Dichlorphenol und 2-Brompentanol als Ausgangsstoffe eingesetzt werden, läßt sich das vorstehende Verfahren (f) durch die folgende Gleichung darstellen:

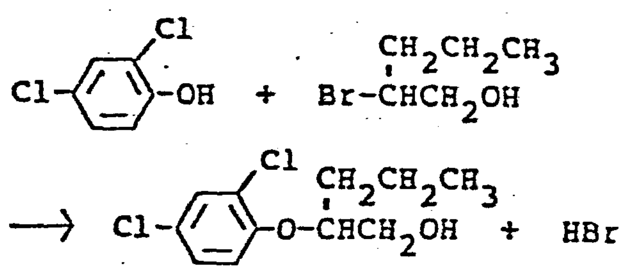

Die meisten der als Ausgangsstoffe in Verfahren (e) eingesetzten Verbindungen der Formel (VIII) sind bekannte Verbindungen, die beispielsweise in J. Am. Chem. Soc. 73 (1951), Seiten 1660 und 4458, beschrieben sind.

Als Beispiele seien genannt:

2-(2-Chlorphenoxy)valeriansäure,
2-(4-Chlorphenoxy)buttersäure,
2-(4-Tolyloxy)buttersäure,
2-(4-Chlorphenoxy)valeriansäure,
2-(4-Methoxyphenoxy)buttersäure,
2-(2,4-Dichlorphenoxy)buttersäure,
2-(2,4-Dichlorphenoxy)valeriansäure,
2-(2,4-Dichlorphenoxy)isovaleriansäure,
2-(2,4-Dichlorphenoxy)capronsäure,
2-(2,4-Dichlorphenoxy)-3,3-dimethylbuttersäure,
2-(2,6-Dichlorphenoxy)buttersäure,
2-[4-(Trifluormethyl)phenoxy]buttersäure,
2-[4-(Trifluormethoxy)phenoxy]buttersäure,
2-[2-Chlor-4-(trifluormethoxy)phenoxy]-buttersäure,
2-[2,6-Dichlor-4-(trifluormethoxy)phenoxy]buttersäure,
2-(4-Nitrophenoxy)-buttersäure,
2-[2,6-Dichlor-4-(trifluormethylthio)phenoxy]buttersäure
und
2-(4-Cyanophenoxy)buttersäure.

Die Säurehalogenide, Alkylester (wie die Methyl- und Ethylester) und Arylester (wie die Phenylester) dieser Verbindungen sind ebenfalls zu nennen.

Die oben beispielhaft genannten Verbindungen der Formel (VIII) können nach allgemein bekannten Synthesemethoden synthetisiert werden, beispielsweise nach der in Acta. Chim. Scand. 8 (1954), Seite 1493, beschriebenen Methode.

Das Verfahren (e) kann beispielsweise in Gegenwart eines inerten Lösungsmittels, etwa von Ethern oder Dioxanen, unter Einsatz von Natriumborhydrid und Lithiumaluminiumhydrid bei einer Reaktionstemperatur von etwa $0^0$ C bis etwa $25^0$ C entsprechend den Angaben in J. Med. Chem. 11 (1968), Seiten 1190-1201, durchgeführt werden.

Die Phenole der Formel (IV) als Ausgangsstoffe in den Verfahren (b) und (f) sind Verbindungen, die auf dem Gebiet der organischen Chemie wohlbekannt sind.

Die meisten der Verbindungen der Formel (IX) sind bekannt. Als Beispiele genannt seien: 2-Brompentanol, 2-Brombutanol, 2-Brom-3-methylbutanol, 2-Bromhexanol und 2-Brom-3,3-dimethylbutanol.

Die im Vorstehenden beispielhaft bezeichneten Verbindungen der Formel (IX) lassen sich in einfacher Weise nach allgemein bekannten Synthesemethoden synthetisieren, beispielsweise nach den in J. Org. Chem. 17 (1952), Seiten 623-628, und J. Am. Chem. Soc. 78 (1956), Seiten 4045-4048 beschriebenen Methoden, und sind beispielsweise in diesen Veröffentlichungen beschrieben.

Das Verfahren (f) läßt sich leicht durchführen, beispielsweise nach der in Chem. Zentralblatt 1915, I, Seite 815, beschriebenen Methode.

Bei der praktischen Durchführung des Verfahrens (f) wird vorzugsweise das Phenol der Formel (IV) bei der Reaktion

mit der Verbindung der Formel (IX) nach vorheriger Überführung in sein Alkalimetall-Salz eingesetzt. Diese Reaktion kann in Gegenwart eines inerten organischen Lösungsmittels (wie Ethanol und Dimethylformamid) durchgeführt werden, wie sie im folgenden beispielhaft aufgeführt werden, oder in Gegenwart eines säurebindenden Mittels.

Zu Beispielen für das Halogenierungsmittel in Verfahren (c) zählen Bromwasserstoffsäure, Phosphortrichlorid, Phosphortribromid und Thionylchlorid.

Das Verfahren (c) ist ein allgemein bekanntes Syntheseverfahren und kann beispielsweise nach der in J. Med. Chem. 11 (1968), Seiten 1190-1201, beschriebenen Methode durchgeführt werden.

Das Verfharen (d) ist ein allgemein bekanntes Syntheseverfahren und kann beispielsweise nach der in J. Med. Chem. 19, Seiten 1333-1336, beschriebenen Methode durchgeführt werden.

Die weiterhin als Ausgangsstoffe in dem erfindungsgemäßen Verfahren (a) eingesetzten Verbindungen der Formel (III) sind auf dem Gebiet der organischen Chemie bekannt. In der Formel (III) steht M vorzugsweise für Wasserstoff, Natrium oder Kalium.

Bei der praktischen Durchführung des Verfahrens (a) können geeignete Verdünnungsmittel sämtliche inerten organischen Lösungsmittel oder auch Wasser sein.

Beispiele für solche Lösungsmittel oder Verdünnungsmittel umfassen Wasser; aliphatische, alicyclische und aromatische Kohlenwasserstoffe (die gegebenenfalls chloriert sein können) wie Hexan, Cyclohexan, Petrolether, Ligroin, Benzol, Toluol, Xylol, Methylenchlorid, Chloroform, Kohlenstofftetrachlorid, Ethylenchlorid, Trichlorethylen und Chlorbenzol; Ether wie Diethylether, Methylethylether, Di-isopropylether, Dibutylether, Propylenoxid, Dioxan und Tetrahydrofuran; Ketone wie Aceton, Methylethylketon, Methylisopropylketon und Methylisobutylketon; Nitrile wie Acetonitril, Propionitril und Acrylnitril; Alkohole wie Methanol, Ethanol, Isopropanol, Butanol und Ethylenglycol; Ester wie Ethylacetat und Amylacetat; Säureamide wie Dimethylformamid und Dimethylacetamid; Sulfone und Sulfoxide wie Dimethylsulfoxid und Sulfolan; sowie Basen wie Pyridin.

Das erfindungsgemäße Verfahren (a) kann in Gegenwart eines säurebindenden Mittels durchgeführt werden. Zu Beispielen für die säurebindenden Mittel zählen die Hydride, Hydroxide, Carbonate, Hydrogencarbonate und Alkoholate von Alkalimetallen sowie tertiäre Amine wie Triethylamin, Diethylanilin und Pyridin, die allgemein verwendet werden. Die Reaktion kann in wirksamer Weise unter Einsatz von Natriumhydrid als Base und Überführung der Verbindung der Formel (III) in ihr Natrium-Salze durchgeführt werden. Ein Erdalkalimetall-Salz der Verbindung der Formel (III) kann in der Reaktion ebenfalls verwendet werden.

Das Verfahren (a) kann innerhalb eines weiten Temperatur-Bereichs, beispielsweise bei einer Temperatur zwischen etwa -20°C und dem Siedepunkt der Mischung, vorzugsweise etwa 20°C und etwa 160°C, durchgeführt werden. Zweckmäßigerweise wird die Reaktion unter normalem atmosphärischen Druck durchgeführt, jedoch ist es möglich, das Verfahren unter erhöhtem oder vermindertem Druck durchzuführen.

Bei der Durchführung des Verfahrens (a) können die gewünschten neuen Verbindungen der Formel (I) beispielsweise dadurch erhalten werden, daß 1 mol der Verbindungen der Formel (II) mit etwa 0,9 bis 1,2 mol, vorzugsweise etwa 0,95 mol bis etwa 1,1 mol, der Verbindungen der Formel (III) umgesetzt wird.

Die bei der Durchführung des erfindungsgemäßen Verfahrens (b) als Reaktionskomponenten benötigten Verbindungen sind durch die Formel (V) definiert. In dieser Formel hat R vorzugsweise diejenigen Bedeutungen, die bereits im Zusammenhang mit der Beschreibung der erfindungsgemäßen Stoffe der Formel (I) vorzugsewise für diesen Rest genannt wurden.

Y hat vorzugsweise diejenigen Bedeutungen, die bereits im Zusammenhang mit der Beschreibung der Verbindungen der Formel (II) vorzugsweise für diesen Rest genannt wurden.

Die Verbindungen der Formel (V) sind bekannt oder lassen sich in einfacher Weise nach üblichen Methoden herstellen.

Das erfindungsgemäße Verfahren (b) wird unter den für derartige Umsetzungen üblichen Bedingungen durchgeführt. Im allgemeinen entsprechen die Umsetzungsbedingungen bei dem erfindungsgemäßen Verfahren (b) denjenigen des Verfahrens (a).

Die nach den erfindungsgemäßen Verfahren erhältlichen Verbindungen der Formel (I) können in Säureadditions-Salze überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der allgemeinen Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Bei der praktischen Durchführung der Verfahren (c) und (d) können die für Verfahren (a) beispielhaft

genannten inerten organischen Lösungsmittel mit Ausnahme von Wasser und Alkoholen als Beispiele für geeignete Verdünnungsmittel genannt werden. In der Praxis des Verfahrens (d) kann in die Reaktion in Gegenwart der säurebindenden Mittel durchgeführt werden, die im Vorstehenden für Verfahren (a) beispielhaft genannt sind.

Das Verfahren (c) kann im wesentlichen innerhalb eines weiten Temperatur-Bereichs, beispielsweise bei einer Temperatur zwischen etwa -30°C und etwa 120°C, vorzugsweise etwa -10°C und etwa 100°C, durchgeführt werden.

Vorzugsweise wird das Verfahren (c) unter normalem atmosphärischen Druck durchgeführt, jedoch ist es auch möglich, unter erhöhtem oder vermindertem Druck zu arbeiten.

Das Verfahren (d) kann beispielsweise bei einer Temperatur in dem Bereich von etwa -20°C bis etwa 100°C, vorzugsweise etwa 0°C bis etwa 50°C, durchgeführt werden.

Vorzugsweise wird das Verfahren (d) unter normalem atmosphärischen Druck durchgeführt, jedoch ist es auch möglich, unter erhöhtem oder vermindertem Druck zu arbeiten.

In der Praxis des Verfahrens (c) können die Verbindungen der Formel (II-a) leicht nach einer üblichen Methode durch Umsetzung der Verbindungen der Formel (VI) mit einem geeigneten Halogenierungsmittel wie den oben genannten erhalten werden.

In der Praxis des Verfahrens (d) können die Verbindungen der Formel (II-b) dadurch erhalten werden, daß 1 mol der Verbindungen der Formel (VI) mit, beispielsweise, etwa 1 bis 1,5 mol, vorzugsweise 1 bis etwa 1,2 mol, der Verbindungen der Formel (VII) umgesetzt wird.

Die erfindungsgemäßen Verbindungen zeigen stark ausgeprägte mikrobizide Wirkung und können in der Praxis zur Bekämpfung unerwünschter Mikroorganismen eingesetzt werden. Die aktiven Verbindungen sind zur Verwendung als Pflanzenschutzmittel geeignet.

Fungizide Mittel werden im Pflanzenschutz eingesetzt zur Bekämpfung von

Plasmodiophoromycetes,
Oomycetes,
Chytridiomycetes,
Zygomycetes,
Ascomycetes,
Basidiomycetes und
Deuteromycetes,

Bakterizide Mittel werden im Pflanzenschutz eingesetzt zur Bekämpfung von

Pseudomonadaceae,
Rhizobiaceae,
Enterobacteriaceae,
Corynebacteriaceae und
Streptomycetaceae.

Einige der Organismen, die fungale und bakterielle Erkrankungen verursachen und unter die oben aufgeführten Sammelbegriffe fallen, seien im Folgenden als nichtbeschränkende Beispiele genannt:

Species Xanthomonas
wie beispielsweise Xanthomonas campestris pv. oryzae;
Species Pseudomonas
wie beispielsweise Pseudomonas syringae pv. lacrymans;
Species Erwinia
wie beispielsweise Erwinia amylovora;
Species Pythium
wie beispielsweise Pythium ultimum;
Species Phytophthora
wie beispielsweise Phytophthora infestans;

Species Pseudoperonospora
wie beispielsweise Pseudoperonospora cubense;
Species Plasmopara
wie beispielsweise Plasmopara viticola;
Species Peronospora
wie beispielsweise Peronospora pisi oder P. brassicae;
Species Erysiphe
wie beispielsweise Erysiphe graminis;
Species Sphaerotheca
wie beispielsweise Sphaerotheca fuliginea;
Species Podosphaera
wie beispielsweise Podosphaera leucotricha;
Species Venturia
wie beispielsweise Venturia inaequalis;
Species Pyrenophora
wie beispielsweise Pyrenophora teres oder P. graminea (Conidiale Form: Drechslera, Synonym: Helminthosporium);
Species Cochliobolus
wie beispielsweise Cochliobolus sativus (Conidiale Form: Drechslera, Synonym: Helminthosporium);
Species Uromyces
wie beispielsweise Uromyces appendiculatus;
Species Puccinia
wie beispielsweise Puccinia recondita;
Species Tilletia
wie beispielsweise Tilletia caries;
Species Ustilago
wie beispielsweise Ustilago nuda oder Ustilago avenae;
Species Pellicularia
wie beispielsweise Pellicularia sasakii;
Species Piricularia
wie beispielsweise Piricularia oryzae;
Species Fusarium
wie beispielsweise Fusarium culmorum;;

Species Botrytis
wie beispielsweise Botrytis cinerea;
Species Septoria
wie beispielsweise Septoria nodorum;
Species Leptosphaeria
wie beispielsweise Leptosphaeria nodorum;
Species Cercospora
wie beispielsweise Cercospora canescens;
Species Alternaria
wie beispielsweise Alternaria brassicae;
Species Pseudocercosporella
wie beispielsweise Pseudocercosporella herpotrichoides.

Insbesondere zählen zu Beispielen für Pflanzenerkrankungen, die gemäß der vorliegenden Erfindung bekämpft werden können:
Pulver-Mehltau (Sphaerotheca fuliginea) der Gurke,
Bohnenrost (Uromyces appendiculatus der (Vits-)Bohne,
Braunfleckenkrankheit (Cochliobolus miyabeanus) von Reis und
"Bakanae"-Krankheit von Reis(Gibberella fujikuroi).

Die gute Tolerierung der aktiven Verbindungen durch die Pflanzen bei den zur Bekämpfung der Pflanzenerkrankungen erforderlichen Konzentrationen erlaubt die Behandlung der oberirdischen Pflanzenteile, der Teile der vegetativen Fortpflanzung und der Samen sowie des Bodens.

Die erfindungsgemäßen Verbindungen können in gebräuchliche Formulierungen überführt werden, etwa Lösungen, Emulsionen, Suspensionen, Pulver, Schäume, Pasten, Granulat, Aerosol, mit der aktiven Verbindung imprägnierte natürliche und synthetische Stoffe, sehr feine Kapseln in

polymeren Substanzen, Überzugsmassen zur Verwendung auf Saatgut (Beizmittel) sowie Formulierungen für den Einsatz mit Verbrennungseinrichtungen wie Räucherpatronen, Räucherdosen und Räucherschlangen sowie für die kalte Vernebelung und die warme Vernebelung nach dem Ultra-Low-Volume-Verfahren.

Diese Formulierungen können in bekannter Weise hergestellt werden, beispielsweise durch Vermischen der aktiven Verbindungen mit Streckmitteln, das heißt mit flüssigen oder verflüssigten gasförmigen oder festen Verdünnungsmitteln oder Trägern, gegebenenfalls unter Verwendung grenzflächenaktiver Mittel, das heißt von Emulgatoren und/oder Dispergiermitteln und/oder schaumbildenden Mitteln. Bei Verwendung von Wasser als Streckmittel können organische Lösungsmittel beispielsweise als Hilfslösungsmittel verwendet werden.

Als flüssige Lösungsmittel, Verdünnungsmittel oder Träger hauptsächlich geeignet sind aromatische Kohlenwasserstoffe wie Xylol, Toluol oder Alkylnaphthaline, chlorierte aromatische oder chlorierte aliphatische Kohlenwasserstoffe wie Chlorbenzole, Chloroethylene oder Methylenchlorid, aliphatische Kohlenwasserstoffe wie Cyclohexan oder Paraffine, beispielsweise Mineralöl-Fraktionen, Alkohole wie Butanol oder Glycol sowie deren Ether und Ester, Ketone wie Aceton, Methylethylketon, Methylisobutylketon oder Cyclohexanon oder stark polare Lösungsmittel wie Dimethylformamid und Dimethylsulfoxid sowie auch Wasser.

Unter verflüssigten gasförmigen Verdünnungsmitteln oder Trägern sind Flüssigkeiten zu verstehen, die bei normaler Temperatur und normalem Druck gasförmig wären,

beispielsweise Aerosol-Treibmittel wie halogenierte Kohlenwasserstoffe und ebenso auch Butan, Propan, Stickstoff und Kohlenstoffdioxid.

Als feste Träger verwendbar sind gemahlene natürliche Minerale wie Kaoline, Tone, Talkum, Kreide, Quarz, Attapulgit, Montmorillonit oder Diatomeenerde und gemahlene synthetische Minerale wie hochdisperse Kieselsäure, Aluminiumoxid und Silicate. Als feste Träger für Granulat können zerkleinerte und fraktionierte Natursteinmaterialien verwendet werden, etwa Calcit, Marmor, Bimsstein, Sepiolith und Dolomit sowie synthetisches Granulat aus anorganischen und organischen Mehlen und Granulat oder organischen Materialien wie Sägemehl, Kokosnußschalen, Maiskolben und Tabakstengel.

Als emulgierende und/oder schaumbildende Mittel können nicht-ionische und anionische Emulgatoren wie Polyoxyethylenfettsäureester, Polyoxyethylenfettalkoholether, beispielsweise Alkylarylpolyglycol-Ether, Alkylsulfonate, Alkylsulfate, Arylsulfonate sowie Albumin-Hydrolyseprodukte verwendet werden. Zu Dispergiermitteln zählen beispielsweise Ligninsulfit-Ablaugen und Methylcellulose.

Haftmittel wie Carboxymethylcellulose und natürliche und synthetische Polymere in Form von Pulvern, Granulat oder Latices wie Gummi arabicum, Polyvinylalkohol und Polyvinylacetat können bei der Formulierung verwendet werden.

Es ist auch möglich, farbgebende Mittel, etwa anorganische Pigmente wie beispielsweise Eisenoxid, Titanoxid

und Preußisch Blau und organische Farbstoffe wie Alizarin-Farbstoffe, Azo-Farbstoffe oder Metallphthalocyanin-Farbstoffe, sowie Spuren-Nährstoffe wie Salze von Eisen, Mangan, Bor, Kupfer, Cobalt, Molybdän und Zink zu verwenden.

Die Formulierungen enthalten im allgemeinen 0,1 bis 95 Gew.-%, vorzugsweise 0,5 bis 90 Gew.-%, der aktiven Verbindung.

Die erfindungsgemäßen Verbindungen können in ihren oben beschriebenen Formulierungen oder ihren verschiedenen Anwendungsformen gemeinsam mit anderen aktiven Verbindungen vorliegen, etwa mit Fungiziden, Bakteriziden, Insektiziden, Akariziden, Nematiziden, Herbiziden, vogelabweisenden Mitteln, Wachstumsfaktoren, Pflanzennährstoffen und Mitteln zur Verbesserung der Bodenstruktur.

Die erfindungsgemäßen Verbindungen können als solche eingesetzt werden, oder sie können in Form solcher Formulierungen oder Anwendungsformen, die daraus durch weitere Verdünnung hergestellt werden, etwa gebrauchsfertigen Lösungen, Emulsionen, Suspensionen, Pulvern, Pasten oder Granulat, eingesetzt werden. Sie werden in üblicher Weise ausgebracht, etwa durch Bewässern, Spritzen, Zerstäuben, Vernebeln, Verdampfen, Einspritzen, Bilden von Aufschlämmungen, Aufpinseln, Streuen, Trockenbedecken, Feuchtbedecken, Naßbedecken, Schlammbedecken und Umhüllen mit einer Kruste.

Bei der Behandlung von Pflanzenteilen können die Konzentrationen der erfindungsgemäßen Verbindung in den Anwendungsformen innerhalb eines beträchtlichen Bereichs variiert

werden. Sie liegen im allgemeinen in einem Bereich von 1 bis 0,0001 Gew.-%, und vorzugsweise von 0,5 bis 0,001 Gew.-%.

Im Fall der Saatgut-Behandlung werden Mengen der erfindungsgemäßen Verbindung von beispielsweise 0,001 bis 50 g, vorzugsweise 0,01 bis 10 g, auf 1 kg des Saatguts zu Anwendung gebracht.

Zur Behandlung des Bodens werden im allgemeinen am Wirkungsort 0,00001 bis etwa 0,1 Gew.-%, insbesondere 0,0001 bis etwa 0,02 Gew.-%, aufgebracht.

Die folgenden Beispiele erläutern die vorliegende Erfindung im einzelnen. Die Erfindung ist jedoch nicht auf diese Beispiele beschränkt.

## Herstellungsbeispiele

### Beispiel 1

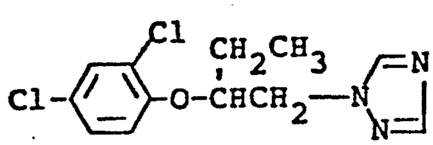

(Verbindung Nr. 1)

Natriumhydrid (0,53 g) wurde in trockenem Dimethylformamid (20 ml) suspendiert, und unter Eiskühlung wurde 1,2,4-Triazol (1,52 g) nach und nach hinzugefügt. Die Mischung wurde 1 h bei Raumtemperatur gerührt, und 2-(2,4-Dichlorphenoxy)-n-butylmethansulfonat (6,26 g) wurden tropfenweise bei weniger als 20°C hinzugefügt.

Die Reaktionsmischung wurde 6 h bei 60°C und dann 3 h unter Rückfluß gerührt. Nach Vervollständigung der Reaktion wurde die Reaktionsmischung zu Eiswasser gegeben, und danach wurde mit Dichlormethan extrahiert. Die Dichlormethan-Lösung wurde mit Wasser gewaschen und getrocknet. Das Lösungsmittel wurde unter vermindertem Druck abgedampft, wonach 1-/‾2-(2,4-Dichlorphenoxy)butyl_7-1H-1,2,4-triazol (5,53 g) erhalten wurde; $n_D^{20}$ = 1,5555.

Beispiel 2

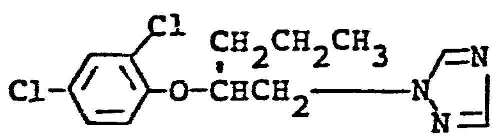

(Verbindung Nr. 2)

Zu Aceton (30 ml) wurden 1-/‾1- Chlormethyl)butoxy7-2,4-dichlorbenzol (5,35 g), 1,2,4-Triazol (1,52 g) und wasserfreies Kaliumcarbonat (3,31 g) hinzugefügt, und die Mischung wurde 3 h bei Raumtemperatur und dann 5 h unter Rückfluß gerührt. Nach Vervollständigung der Reaktion wurde das Aceton unter vermindertem Druck abgedampft. Der Rückstand wurde mit Wasser versetzt und mit Toluol extrahiert. Der Toluol-Extrakt wurde mit Wasser gewaschen und getrocknet. Das Lösungsmittel wurde unter vermindertem Druck abgedampft, wonach 1-/‾2-(2,4-Dichlorphenoxy) -n-pentyl_7-1H-1,2,4-triazol (5,34 g) erhalten wurde; $n_D^{20}$ = 1,5480.

Tabelle 1 zeigt Verbindungen der Formel (I) gemäß der vorliegenden Erfindung, die nach den gleichen Methoden wie in den Beispielen 1 und 2 hergestellt wurden.

Tabelle 1

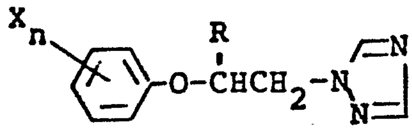

(I)

| Verbindung Nr. | $X_n$ | R | |
|---|---|---|---|
| 3 | - | $-C_2H_5$ | |
| 4 | $4-CH_3$ | $-C_2H_5$ | $n_D^{20}$1.5303 |
| 5 | $3,5-(CH_3)_2$ | $-C_2H_5$ | $n_D^{20}$1.5288 |
| 6 | 2-Cl | $-C_3H_7$-n | $n_D^{20}$1.5405 |
| 7 | 4-Cl | $-C_2H_5$ | $n_D^{20}$1.5450 |
| 8 | 4-Cl | $-C_3H_7$-n | $n_D^{20}$1.5386 |
| 9 | $2,4-Cl_2$ | $-CH_3$ | $n_D^{20}$ 1.5620 |
| 10 | $2,4-Cl_2$ | $-C_3H_7$-iso | $n_D^{20}$1.5490 |
| 11 | $2,4-Cl_2$ | $-C_4H_9$-n | $n_D^{20}$1.5442 |
| 12 | $2,4-Cl_2$ | $-C_4H_9$-tert | Schmp.83-86 °C |
| 13 | $2,6-Cl_2$ | $-C_2H_5$ | $n_D^{20}$1.5565 |
| 14 | $4-OCH_3$ | $-C_2H_5$ | $n_D^{20}$1.5350 |
| 15 | $4-CF_3$ | $-C_2H_5$ | $n_D^{20}$1.4882 |
| 16 | $4-OCF_3$ | $-C_2H_5$ | $n_D^{20}$1.4780 |
| 17 | $4-NO_2$ | $-C_2H_5$ | |
| 18 | 4-CN | $-C_2H_5$ | |
| 19 | 2-Cl, $4-OCF_3$ | $-C_2H_5$ | $n_D^{20}$1.4900 |
| 20 | $2,6-Cl_2$, $4-OCF_3$ | $-C_2H_5$ | $n_D^{20}$1.5038 |
| 21 | $2,6-Cl_2$, $4-SCF_3$ | $-C_2H_5$ | $n_D^{20}$1.5362 |

Beispiel 3

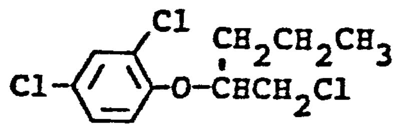

(Verbindung Nr. II-2)

2-(2,4-Dichlorphenoxy)-n-pentanol (24,9 g) und Pyridin (7,9 g) wurden in 80 ml Ether gelöst. Unter Rühren wurden 16,7 g Thionylchlorid tropfenweise bei -10°C zu dieser Mischung hinzugefügt. Die Mischung wurde 1 h bei Raumtemperatur und weiter 1 h unter Rückfluß gerührt. Die Ether-Lösung wurde mit Wasser, einer 5-proz. wäßrigen Kaliumhydroxid-Lösung, 2-proz. Salzsäure und weiter mit Wasser gewaschen und über wasserfreiem Natriumsulfat getrocknet.

Das Lösungsmittel wurde unter vermindertem Druck abgedampft, wonach ein Rohprodukt (25,9 g) erhalten wurde. Destillation des Rohprodukts unter vermindertem Druck lieferte 1-/¯1-Chlormethyl)butoxy7-2,4-dichlorbenzol (13,8 g).

Beispiel 4

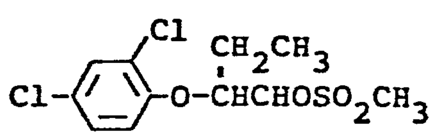

(Verbindung Nr. II-1)

Methansulfonylchlorid (12,60 g) wurde tropfenweise bei einer Temperatur unterhalb von 0°C unter Rühren zu

einer 2-(2,4- Dichlorphenoxy)-n-butanol (23,5 g) und Triethylamin (11,1 g) enthaltenden Lösung in Toluol (150 ml) hinzugefügt. Die Mischung wurde 6 h bei Raumtemperatur gerührt, um die Reaktion zu beenden. Die Reaktionsmischung wurde mit Wasser, 2-proz. Salzsäure, einer 5-proz. wäßrigen Kaliumhydroxid-Lösung und mit Wasser gewaschen und über wasserfreiem Natriumsulfat getrocknet. Das Lösungsmittel wurde unter vermindertem Druck abgedampft, wonach 2-(2,4-Dichlorphenoxy)-n-butyl-methansulfonat (30,9 g) erhalten wurde. $n_D^{20}$ = 1,5310.

Die nachstehende Tabelle 2 zeigt Verbindungen der Formel (II), die nach den gleichen Methoden wie in den Beispielen 3 und 4 hergestellt wurden.

Tabelle 2

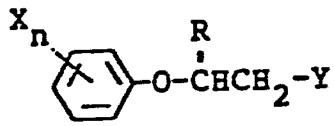 (II)

| Verbindung Nr. | $X_n$ | R | Y | |
|---|---|---|---|---|
| II-1-a | 2,4-$Cl_2$ | $-C_2H_5$ | Cl | |
| II-1-b | 2,4-$Cl_2$ | $-C_2H_5$ | Br | |
| II-2-a | 2,4-$Cl_2$ | $-C_3H_7$-n | $-OSO_2CH_3$ | $n_D^{20}$ 1.5262 |
| II-2-b | 2,4-$Cl_2$ | $-C_3H_7$-n | Br | |
| II-3 | 4-$CH_3$ | $-C_2H_5$ | $-OSO_2CH_3$ | $n_D^{20}$ 1.5071 |
| II-4 | 3,5-$(CH_3)_2$ | $-C_2H_5$ | $-OSO_2CH_3$ | $n_D^{20}$ 1.5073 |
| II-5 | 2-Cl | $-C_3H_7$-n | $-OSO_2CH_3$ | $n_D^{20}$ 1.5162 |
| II-6 | 4-Cl | $-C_2H_5$ | $-OSO_2CH_3$ | $n_D^{20}$ 1.5202 |
| II-7 | 4-Cl | $-C_3H_7$-n | $-OSO_2CH_3$ | $n_D^{20}$ 1.5153 |
| II-8 | 2,4-$Cl_2$ | $-C_3H_7$-iso | $-OSO_2CH_3$ | $n_D^{20}$ 1.5275 |
| II-9 | 2,4-$Cl_2$ | $-C_4H_9$-n | $-OSO_2CH_3$ | $n_D^{20}$ 1.5222 |
| II-10 | 2,4-$Cl_2$ | $-C_4H_9$-tert | $-OSO_2CH_3$ | $n_D^{20}$ 1.5235 |
| II-11 | 2,6-$Cl_2$ | $-C_2H_5$ | $-OSO_2CH_3$ | Schmp.65-66°C |
| II-12 | 4-$OCH_3$ | $-C_2H_5$ | $-OSO_2CH_3$ | $n_D^{20}$ 1.5122 |
| II-13 | 4-$CF_3$ | $-C_2H_5$ | $-OSO_2CH_3$ | $n_D^{20}$ 1.4695 |
| II-14 | 4-$OCF_3$ | $-C_2H_5$ | $-OSO_2CH_3$ | $n_D^{20}$ 1.4604 |
| II-15 | 4-$NO_2$ | $-C_2H_5$ | $-OSO_2CH_3$ | |
| II-16 | 4-CN | $-C_2H_5$ | $-OSO_2CH_3$ | |
| II-17 | 2-Cl, 4-$OCF_3$ | $-C_2H_5$ | $-OSO_2CH_3$ | $n_D^{20}$ 1.4732 |
| II-18 | 2,6-$Cl_2$, 4-$OCF_3$ | $-C_2H_5$ | $-OSO_2CH_3$ | Schmp.77-79° C |
| II-19 | 2,6-$Cl_2$, 4-$SCF_3$ | $-C_2H_5$ | $-OSO_2CH_3$ | Schmp 87-89° C |

0202566

Beispiel 5

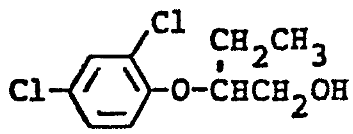

(Verbindung Nr. VI-1)

Lithiumaluminiumhydrid (13,5 g) wurde in trockenem Ether (500 ml) suspendiert, und Ethyl-2-(2,4-Dichlorphenoxy)butyrat (89,3 g) wurde tropfenweise unter Rühren bei 0°C bis 5°C hinzugefügt. Die Mischung wurde 8 h bei Raumtemperatur gerührt, und Wasser wurde allmählich zur Zersetzung des überschüssigen Lithiumaluminiumhydrids hinzugefügt. Die Reaktionsmischung wurde filtriert, und das Filtrat wurde konzentriert, wonach 65,1 g eines Rohprodukts erhalten wurden.

Die Destillation des Rohprodukts unter vermindertem Druck lieferte 2-(2,4-Dichlorphenoxy)-n-butanol (54,2 g); Sdp. 139-140°C/2,67 mbar (2 mmHg).

Beispiel 6

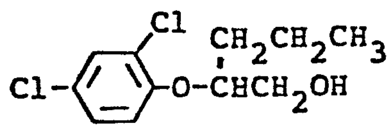

(Verbindung Nr. VI-2)

Zu einer Natrium-Ethylat-Ethanol-Lösung, die aus metallischem Natrium (4,60 g) und wasserfreiem Ethanol (100 ml) hergestellt worden war, wurde 2,4-Dichlorphenol (32,6 g) hinzugefügt. Die Mischung wurde gerührt.

Die Reaktionslösung wurde 4 h bei 60°C und 4 h unter Rückfluß gerührt, um die Reaktion zu beenden. Das Ethanol wurde unter vermindertem Druck abgedampft, und der Rückstand wurde mit Wasser versetzt und dann mit Toluol extrahiert.

Der Toluol-Extrakt wurde mit Wasser, einer 5-proz. wäßrigen Natriumhydroxid-Lösung und weiter mit Wasser gewaschen und über wasserfreiem Natriumsulfat getrocknet.

Das Lösungsmittel wurde unter vermindertem Druck abgedampft, wonach ein Rohprodukt (38,4 g) erhalten wurde. Das Rohprodukt wurde unter vermindertem Druck destilliert, wonach 2-(2,4-Dichlorphenoxy)-n-pentanol (29,3 g) erhalten wurde; Sdp. 147-148°C/2,67 mbar (2 mmHg).

Die nachstehende Tabelle 3 zeigt Verbindungen der Formel (VI), die nach den gleichen Methoden wie in den Beispielen 5 und 6 hergestellt wurden.

Tabelle 3

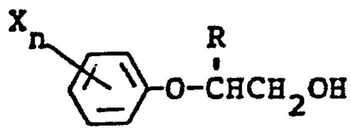 (VI)

| Verbindung Nr. | $X_n$ | R | |
|---|---|---|---|
| VI-3 | $4-CH_3$ | $-C_2H_5$ | $n_D^{20}$ 1.5148 |
| VI-4 | $3,5-(CH_3)_2$ | $-C_2H_5$ | $n_D^{20}$ 1.5145 |
| VI-5 | 2-Cl | $-C_3H_7-n$ | Sdp. 120-122°C/ 1,6 mbar (1,2 mmHg) |
| VI-6 | 4-Cl | $-C_2H_5$ | Sdp. 117-118°C/ 1,6 mbar (1,2 mmHg) |
| VI-7 | 4-Cl | $-C_3H_7-n$ | Sdp. 126-127°C/ 1,6 mbar (1,2 mmHg) |
| VI-8 | $2,4-Cl_2$ | $-C_3H_7-iso$ | $n_D^{20}$ 1.5390 |
| VI-9 | $2,4-Cl_2$ | $-C_4H_9-n$ | Sdp. 139-144°C/ 0,9 mbar (1,2 mmHg) |
| VI-10 | $2,4-Cl_2$ | $-C_4H_9-tert$ | $n_D^{20}$ 1.5344 |
| VI-11 | $2,6-Cl_2$ | $-C_2H_5$ | Sdp. 122-123°C/ 1,6 mbar (1,2 mmHg) |
| VI-12 | $4-OCH_3$ | $-C_2H_5$ | $n_D^{20}$ 1.5215 |
| VI-13 | $4-CF_3$ | $-C_2H_5$ | $n_D^{20}$ 1.4670 |
| VI-14 | $4-OCF_3$ | $-C_2H_5$ | $n_D^{20}$ 1.4560 |
| VI-15 | $4-NO_2$ | $-C_2H_5$ | |
| VI-16 | 4-CN | $-C_2H_5$ | |
| VI-17 | 2-Cl, $4-OCF_3$ | $-C_2H_5$ | $n_D^{20}$ 1.4694 |
| VI-18 | $2,6-Cl_2$, $4-OCF_3$ | $-C_2H_5$ | $n_D^{20}$ 1.4842 |
| VI-19 | $2,6-Cl_2$, $4-SCF_3$ | $-C_2H_5$ | $n_D^{20}$ 1.5210 |

Biologische Test-Beispiele:

Vergleichs-Verbindung A-1:

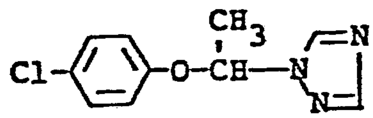

(die in der JP-OS 33 675/1977 beschriebene Verbindung).

Beispiel 7

Test auf Wirksamkeit gegen den Pulver-Mehltau der Gurke

Herstellung einer Chemikalie:

Test-Verbindung: 30 Teile;
Organisches Lösungsmittel (Xylol): 55 Teile;
Emulgator: 8 Teile Polyoxyethylenalkylphenylether und
7 Teile Calciumalkylbenzolsulfonat.

Ein emulgierbares Konzentrat wurde aus den oben angegebenen Ingredienzien hergestellt und mit Wasser verdünnt.

Jede der Test-Verbindungen in Form einer Emulsion wurde mit einer Sprühpistole auf in unglasierten 9 cm-Töpfen gezogene Gurken (Varietät: "Tokiwajibai") im 2-Blatt-Stadium aufgesprüht. Einen Tag nach dem Sprühen wurden die Pflanzen mit einer Sporen-Suspension von Sphaerotheca fuliginea durch Sprühen inokuliert. Die Töpfe wurden in einem Raum mit einer konstanten Temperatur von 23°C stehen gelassen. Zehn Tage später wurde der Grad des Befalls aufgrund des prozentualen Verhältnisses der Läsionen zeigenden Flächen bestimmt. Der Bekämpfungsindex wurde berechnet.

| Grad des Befalls | Fläche der Verletzungen (%) |
|---|---|
| 0 | 0 |
| 0,5 | 2 oder weniger |
| 1 | 3 bis 5 |
| 2 | 6 bis 15 |
| 3 | 16 bis 30 |
| 4 | 31 bis 50 |
| 5 | 51 oder mehr |

$$\text{Bekämpfungs-Index (\%)} = \frac{\text{Grad des Befalls der unbehandelten Fläche} - \text{Grad des Befalls der behandelten Fläche}}{\text{Grad des Befalls der unbehandelten Fläche}} \times 100$$

Die erfindungsgemäßen Verbindungen zeigten eine hervorragende Bekämpfungswirkung bei Wirkstoff-Konzentrationen von 25 ppm oder weniger. Die mit typischen Beispielen erhaltenen Ergebnisse sind in der Tabelle 4 aufgeführt.

Tabelle 4

| Verbindung Nr. | Wirkstoff-Konzentration (ppm) | Bekämpfungs-Index (%) |
|---|---|---|
| 1 | 25 | 100 |
| 2 | 25 | 100 |
| Vergleichs-Verbindung | | |
| A-1 | 25 | 30 |

Beispiel 8

Test zur Bestimmung der Wirksamkeit der Bekämpfung von (Vits-)Bohnenrost

Eine Test-Verbindung in Form einer Emulsion, die wie in Beispiel 7 hergestellt worden war, wurde unter Verwendung einer Spritzpistole auf Vits-Bohnen (Varietät: Shinedogawa) im Stadium der ersten Blatt-Bildung aufgesprüht, die in unglasierten 9 cm-Töpfen angezogen wurden. Einen Tag nach dem Sprühen wurden die Pflanzen mit einer Sporen-Suspension von Uromyces appendiculatus durch Sprühen inokuliert. Die Töpfe wurden in einer thermostatisierten Kammer bei 23°C gehalten. Zehn Tage später wurde der Grad des Befalls aufgrund des nachstehenden prozentualen Verhältnisses der Läsionen zeigenden Flächen bestimmt, und die Bekämpfungswirkung wurde wie in Beispiel 7 berechnet.

| Grad des Befalls | Fläche der Verletzungen (%) |
|---|---|
| 0 | 0 |
| 0,5 | 0,5 |
| 1 | 2 |
| 2 | 5 |
| 3 | 10 |
| 4 | 20 |
| 5 | 30 oder mehr |

$$\text{Bekämpfungs-Index (\%)} = \frac{\text{Grad des Befalls der unbehandelten Fläche} - \text{Grad des Befalls der behandelten Fläche}}{\text{Grad des Befalls der unbehandelten Fläche}} \times 100$$

Die erfindungsgemäßen Verbindungen zeigten eine hervorragende Bekämpfungswirkung bei Wirkstoff-Konzentrationen von 25 ppm oder weniger. Die mit typischen Beispielen erhaltenen Ergebnisse sind in der Tabelle 5 aufgeführt.

Tabelle 5

| Verbindung Nr. | Wirkstoff-Konzentration (ppm) | Bekämpfungs-Index (%) |
|---|---|---|
| 1 | 25 | 100 |
| 2 | 25 | 100 |
| Vergleichs-Verbindung | | |
| A-1 | 25 | 40 |

Patentansprüche

1. Phenoxyalkyltriazole der Formel

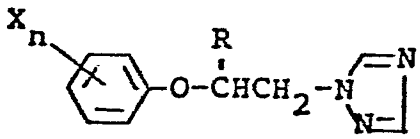

(I)

in welcher

R für Alkyl steht,

X für Halogen, Alkyl, Alkoxy, Alkylthio, Halogenalkyl, Halogenalkoxy, Halogenalkylthio, Nitro und/oder Cyano steht und

n für die Zahlen 0, 1, 2, 3, 4, oder 5 steht,

sowie deren Säureadditions-Salze.

2. Verbindungen gemäß Anspruch 1, dadurch gekennzeichnet, daß

R für Alkyl mit 2 bis 5 Kohlenstoffatomen steht,

X gleichartig oder verschieden ist und für Fluor, Chlor, Brom, Alkyl mit 1 bis 4 Kohlenstoffatomen, Alkoxy mit 1 bis 4 Kohlenstoffatomen, Alkylthio mit 1 bis 4 Kohlenstoffatomen,

gegebenenfalls durch Fluor und/oder Chlor substituiertes Alkyl mit 1 bis 3 Kohlenstoffatomen, gegebenenfalls durch Fluor und/oder Chlor substituiertes Alkoxy mit 1 bis 3 Kohlenstoffatomen, gegebenenfalls durch Fluor und/oder Chlor substituiertes Alkylthio mit 1 bis 3 Kohlenstoffatomen, Nitro und/oder Cyan steht und

n für die Zahlen 1, 2 oder 3 steht.

3. Verbindungen gemäß Anspruch 1, dadurch gekennzeichnet, daß

R für Ethyl, n-Propyl, Isopropyl, n-Butyl, Isobutyl, sec-Butyl oder t-Butyl steht,

X gleichartig oder verschieden ist und für Fluor, Chlor, Methyl, Ethyl, Methoxy, Ethoxy, Methylthio, Trifluormethyl, Trifluormethoxy, Trifluormethylthio, Nitro und/oder Cyano steht und

n für 1 oder 2 steht.

4. Verbindung gemäß Anspruch 1, gekennzeichnet durch die Formel

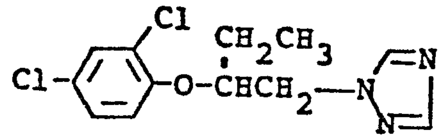

5. Verbindung gemäß Anspruch 1, gekennzeichnet durch die Formel

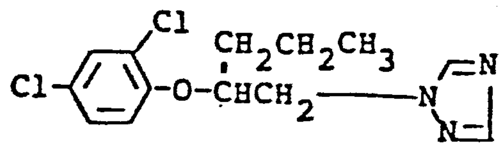

6. Verfahren zur Herstellung von Phenoxyalkyltriazolen der Formel

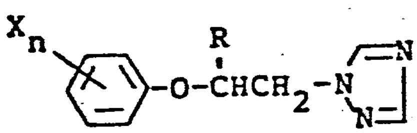

(I)

in welcher

R für Alkyl steht,

X für Halogen, Alkyl, Alkoxy, Alkylthio, Halogenalkyl, Halogenalkoxy, Halogenalkylthio, Nitro und/oder Cyano steht und

n für die Zahlen 0, 1, 2, 3, 4, oder 5 steht,

sowie von deren Säureadditions-Salzen,

dadurch gekennzeichnet, daß man

a) Verbindungen der Formel

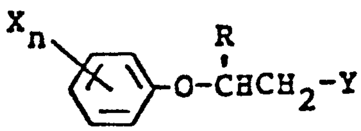

(II)

in welcher

R, X und n die oben angegebene Bedeutung haben und

Y für Halogen oder eine Gruppe der Formel $-SO_2R^1$ steht, worin

$R^1$ für Alkyl oder gegebenenfalls substituiertes Alkyl steht,

mit Verbindungen der Formel

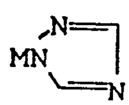

(III)

in welcher

M für Wasserstoff oder ein Alkalimetallatom steht,

gegebenenfalls in Gegenwart eines inerten Verdünnungsmittels und gegebenenfalls in Gegenwart eines Säurebindemittels umsetzt,

oder

b) Verbindungen der Formel

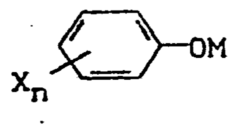 (IV)

in welcher

X, M und n die oben angegebene Bedeutung haben,

mit Verbindungen der Formel

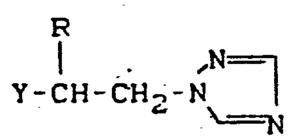 (V)

in welcher

R und Y die oben angegebene Bedeutung haben,

gegebenenfalls in Gegenwart eines inerten Lösungsmittels sowie gegebenenfalls in Gegenwart eines Säurebindemittels umsetzt, wobei die Verbindungen der Formel (I) auch in Form ihrer Säureadditions-Salze eingesetzt werden können,

und gegebenenfalls anschließend an die so erhaltenen Stoffe der Formel (I) eine Säure addiert.

7. Fungizide Mittel, gekennzeichnet durch einen Gehalt an mindestens einem Phenoxyalkyltriazol der Formel (I) bzw. an einem Säureadditions-Salz eines Phenoxyalkyltriazols der Formel (I).

8. Verfahren zur Bekämpfung von Pilzen, dadurch gekennzeichnet, daß man Phenoxyalkyltriazole der Formel (I) oder deren Säureadditions-Salze auf Pilze und/oder deren Lebensraum einwirken läßt.

9. Verwendung von Phenoxyalkyltriazolen der Formel (I) bzw. von deren Säureadditions-Salzen zur Bekämpfung von Pilzen.

10. Verfahren zur Herstellung von fungiziden Mitteln, dadurch gekennzeichnet, daß man Phenoxytriazole der Formel (I) bzw. deren Säureadditions-Salze mit Streckmitteln und/oder oberflächenaktiven Mitteln vermischt.